Europäisches Patentamt

European Patent Office

Office européen des brevets

(19)

(11) Veröffentlichungsnummer: **0 137 301**
**B1**

(12) **EUROPÄISCHE PATENTSCHRIFT**

(45) Veröffentlichungstag der Patentschrifft:
28.02.90

(21) Anmeldenummer: 84110535.6

(22) Anmeldetag: 05.09.84

(51) Int. Cl.⁵: **C 12 N 9/04, C 12 P 7/42 //**
**(C12N9/04, C12R1:38)**

(54) 2-Oxocarbonsäurereduktase, ihre Herstellung und Verwendung.

(30) Priorität: 09.09.83 DE 3332562

(43) Veröffentlichungstag der Anmeldung:
17.04.85 Patentblatt 85/16

(45) Bekanntmachung des Hinweises auf die Patenterteilung:
28.02.90 Patentblatt 90/09

(84) Bennante Vertragsstaaten:
AT BE CH DE FR GB IT LI NL

(56) Entgegenhaltungen:
STUD. ORG. CHEM. AMSTERDAM (BIOMIMETIC CHEM.) Band 13, Juli 1983, Seiten 207-227; H.SIMON et al.: "Chiral synthons by biohydrogenation or electroenzymatic reduction"
CHEMICAL ABSTRACTS, Band 98, Nr. 13, 28.März 1983, Seite 437, Zusamm Nr. 105259t, Columbus, Ohio, US; S.R.SARKAR: "NAD and NADP independent lactate oxidoreductase in Schwartz leukemia ascites cells", & ACTA. BIOL. MED. GER. 1982, 41(11), 1085-6
CHEMICAL ABSTRACTS, Band 84, Nr. 9, 1. März 1976, Seite 210, Zusammenfassung Nr. 55732e, Columbus, Ohio, US; T.EBISUNO et al.: "D-alpha-Hydroxyglutarate dehydrogenase of rhodospirillum rubrum", & J. BIOCHEM. (TOKYO) 1975, 78(6), 1321-9
FEBS LETTERS, Band 167, Nr. 1, Februar 1984, Seiten 29-32, Federation of European Biochemical Societies; S.NEUMANN et al.: "On a non-pyridine nucleotide-dependent 2-oxo-acid reductase of broad substrate specificity from two proteus species"

(73) Patentinhaber: BASF Aktiengesellschaft
Carl-Bosch-Strasse 38
D-6700 Ludwigshafen (DE)

(72) Erfinder: Guenther, Helmut, Dr.
Amperblick 11
D-8051 Haag (DE)
Erfinder: Neumann, Stefan
Frühlingstrasse 8
D-8044 Unterschleissheim (DE)
Erfinder: Simon, Helmut, Prof. Dr.
Egilbertstrasse 31
D-8050 Freising (DE)

## Beschreibung

Die Erfindung betrifft eine neue 2-Oxocarbonsäurereduktase, deren Herstellung sowie deren Verwendung zur Reduktion von 2-Oxocarbonsäuren.

Es ist bereits bekannt, 2-Oxocarbonsäuren enzymatisch zu reduzieren. Für diese Reduktionen werden Enzyme verwendet, die als Cosubstrate Pyridinnukleotide benötigen. Als Beispiel für eine solche Reaktion sei die Herstellung von L-Lactat aus Pyruvat genannt (J.Org.Chem. *47*, 2816 (1982)). Es ist weiter bekannt, aus racemischen Gemischen von 2-Hydroxycarbonsäuren durch enantioselektiven, mikrobiellen Abbau optisch reine 2-Hydroxycarbonsäuren herzustellen (Appl. Environ. Microbiol. *45*, 884 (1983)). Die enzymatische Reduktion mit Pyridinnukleotid-abhängigen Reduktasen hat jedoch den Nachteil, daß noch ein zweites Enzym benötigt wird, welches das oxidierte Pyridinnukleotid wieder regeneriert. Hinzu kommt, daß die Pyridinnukleotide bei längerem Einsatz zerfallen.

Es wurde nun ein Enzym gefunden, welches diese Nachteile nicht besitzt.

Gegenstand der Erfindung ist eine pyridinnukleotid-unabhängige und keine Flavingruppen enthaltende 2-Oxocarbonsäurereduktase.

Die Oxocarbonsäurereduktase besitzt ein Molekulargewicht von etwa 500.000. Ihr isoelektrischer Punkt liegt bei 4,9. Ihr nutzbarer pH-Bereich liegt zwischen 6 und 8 mit einem Maximum bei 6,8. Die Temperaturabhängigkeit der Aktivität des Enzyms zeigt die folgende Tabelle. Bezugswert ist die Aktivität bei 24°C, die gleich 100 % gesetzt wurde.

### Tabelle 1

| T (°C) | Aktivität (%) | T (°C) | Aktivität (%) |
|--------|---------------|--------|---------------|
| 14 | 46 | 34 | 220 |
| 19 | 72 | 39 | 306 |
| 24 | =100 | 44 | 368 |
| 29 | 150 | 49 | 440 |

Bei 45°C zeigt das oxidierte Enzym im Phosphatpuffer in Gegenwart von Tetracyclin eine Halbwertszeit von etwa 13 h. Bei 35° ist diese Zeit ca. 55 h und im reduzierten Zustand in Gegenwart von reduziertem Methylviologen ca. 100 h. Der Aktivitätsverlauf ist nicht 1. Ordnung. Nach 8 bzw. 40 h ist beim oxidierten Enzym noch keine Aktivitätsabnahme zu beobachten. Durch EDTA wird die Aktivität des Enzyms nicht beeinflußt.

Als einzige prosthetische Gruppe konnte bislang säurelabiler Schwefel festgestellt werden, der vermutlich von Fe/S-Clustern herrührt. Flavingruppen lassen sich spektralphotometrisch nicht nachweisen.

Besonders interessant ist, daß die Aktivität des Enzyms durch oxidiertes Methylviologen gesteigert werden kann. In Gegenwart von steigenden Konzentrationen von MV$^{++}$ und 0,15 mM MV$^+$ verhalten sich die Initialgeschwindigkeiten wie folgt:

| | U/ml | |
|---|---|---|
| MV$^{++}$ mM | P. mirabilis | P. vulgris |
| 0,01 | 18,1 | 13,3 |
| 0,08 | 41,9 | 24,4 |
| 0,16 | 46,0 | 31,3 |
| 0,75 | 62,0 | 41,5 |

(In einer 2 mm Küvette waren 0,3 ml 0,1 M Trisacetat Puffer pH 7,0, 2μMol 2-Oxo-4-methylpentanoat, jeweils die gleiche Enzymmenge sowie 0,2 mM reduziertes Methylviologen und die in der Tab. angegebenen Konzentrationen an oxidiertem Methylviologen inkubiert.)

Die 2-Oxocarbonsäurereduktase wird erhalten, indem man einen das Enzym enthaltenden Mikroorganismus, z. B. Proteus vulgaris DSM 30 118 oder Proteus mirabilis DSM 30 115, mechanisch aufschließt, zentrifugiert, den Überstand ultrazentrifugiert, den Niederschlag in einer Detergenzlösung aufschlämmt und feste Verunreinigungen durch erneute Ultrazentrifugation enfernt.

Die erste Zentrifugation dient zur Entfernung grober Verunreinigungen. Sie wird in der Regel bei 5000 bis 20 000 g 5 bis 10 min lang durchgeführt. Der Niederschlag wird verworfen. Der Überstand wird anschließend bei 0 bis 10°C und 80 000 bis 120 000 g 1 bis 2 h zentrifugiert. Der so erhaltene Niederschlag wird in kaltem Puffer aufgeschlämmt und erneut bei 80 000 bis 120 000 g zentrifugiert. Der Niederschlag wird mit einem Dispergiermittel - wie $^R$Triton X 100 oder $^R$Tween 80 - und einem Puffer (pH 6 - 8) versetzt und wiederum bei 80 000 bis 120 000 g zentrifugiert. Der so erhaltene Überstand enthält das Enzym in gelöster Form.

Mit Hilfe des neuen Enzyms lassen sich überraschend viele 2-Oxocarbonsäuren und deren Ester stereospezifisch zu den entsprechenden (2R)-Hydroxycarbonsäuren bzw. deren Estern reduzieren. Beispiele für solche Säuren sind: 2-Oxopropansäure (= Brenztraubensäure), 3-Fluor-2-oxopropansäure, 3-Methyl-2-oxopentansäure (beide enantiomere Formen), 4-Methyl-2-oxopentansäure, Phenylketoessigsäure, Phenylbrenztraubensäure, 5-Benzyloxy-ß-indolylbrenztraubensäure, Ketobernsteinsäure, 2-Oxoglutarsäure, 2-Oxoadipinsäure, 2-Oxoazelainsäure, 2-Oxosebazinsäure, 2-Oxoundecandicarbonsäure, 2-Oxo-4-(methylthio)buttersäure, Indolylbrenztraubensäure, 2-Oxo-4-methylphosphinobuttersäure. Ein Beispiel für einen Ester ist der Brenztraubensäure-ethylester. Die Reduktionsprodukte sind in der Regel zu über 98 % optisch rein. Teilweise liegt die optische Reinheit sogar über 99,5 %.

Weiter benötigt das neue Enzym keine reduzierten Pyridinnukleotide für seine Wirksamkeit. Es kann Elektronen direkt von einem Elektronenträger — wie Methyl- oder Benzylviologen in reduziertem Zustand — auf das Substrat übertragen.

## Beispiel 1

Proteus mirabilis (DSM 30 115) wurde in einem Vollmedium (Hefeextrakt 5,0 g, Glucose 5,0 g, Pepton aus Fleisch 20,0 g, $K_2HPO_4$ 5,0 g, $H_2O$ ad 1000 ml, pH 7,2) bei 35°C unter Begasung mit einem Gemisch von 97 % $N_2$ und 3 % $O_2$ gezüchtet. Höhere Enzymaktivitäten im Rohextrakt werden mit *Proteus vulgaris* (DSM 30118) erhalten. In diesem Fall muß jedoch unter völligem Sauerstoffausschluß angezüchtet werden. Im Rohextrakt lassen sich spezifische Enzymaktivitäten von 7 - 9 U/mg Protein erhalten. Die so erhaltenen Zellen wurden in der frühen stationären Phase geerntet. 1 Gewichtsteil Bakterienfeuchtmasse wurde in 3 Teilen 20 mM Kaliumphosphatpuffer pH 7,0 aufgeschlämmt und in einem Eisbad unter $N_2$ Atmosphäre mit einem Ultraschallgerät beschallt, bis mehr als 90 % der Zellen aufgeschlossen waren, was mikroskopisch festgestellt wurde.

64 ml des so erhaltenen Rohextrakts wurden 10 min bei 10 000 g und 4°C zentrifugiert und der Niederschlag verworfen. Der Überstand wurde 90 min bei 100 000 g und 4°C zentrifugiert und der Überstand verworfen. Der Niederschlag wurde mit 32 ml 20 mM Kaliumphosphatpuffer pH 7,0 gewaschen und nochmals 60 min bei 100 000 g zentrifugiert. Der Überstand wurde wiederum verworfen. Der Niederschlag wurde in 32 ml Puffer 20 ml 20 mM Kaliumphosphatpuffer pH 7,0 suspendiert, der 0,2 g (R)Triton X-100 enthielt. Nach 45 min Rühren wurde 60 min bei 100 000 g bei 0°C zentrifugiert. Die Reduktase war im Überstand. Die Ausbeute betrug 80 %.

## Beispiel 2

10 ml des gemäß Beispiel 1 erhaltenen Rohextrakts mit insgesamt 300 U (spez.Akt. 1,3 U/mg Protein) wurden auf eine (R)Sephacryl S-1000 Säule (Bettvolumen 425 ml) aufgetragen und mit 20 mM Kaliumphosphatpuffer pH 7.0 bei einer Flußrate von 30 ml/h bei 4°C eluiert. Das Enzym erschien in Verbindung mit Membranpartikeln nach ca. 6 h. Die die Aktivität enthaltenden Fraktionen (55 ml) wurden vereinigt und durch Ultrafiltration auf 7 ml eingeengt.

Diese 7 ml erhielten 90 % des Enzyms des Rohextrakts mit einer spezifischen Aktivität von 24 U/mg.

## Beispiel 3

50 g Bakterienfeuchtmasse wurde analog Beispiel 1 aufgeschlossen. Das Volumen des Überstandes der letzten Zentrifugation wurde durch Ultrafiltration auf 11 ml eingeengt (500 U/ml Reduktase und 37 mg Protein/ml), auf eine (R)Sepharose 6B-Säule (Volumen 440 ml) aufgetragen und mit einer Lösung von 20 mM Kaliumphosphatpuffer pH 7,0, 0,05 % (R)Triton X 100 und 1 mM Dithioerythrit eluiert. Die vereinigten Enzym-enthaltenden Fraktionen wurden durch Ultrafiltration auf 35 ml eingeengt. Durch abermalige Ultrafiltration nach Verdünnung wurden 35 ml einer Lösung erhalten, die 10 mM an Kaliumphosphatpuffer war. Nach Auftragen auf eine Hydroxyapatitsäule (Bio Gel HTP) mit 100 ml Bettvolumen, die mit 10 mM Phosphatpuffer, 0,05 % (R)Triton X 100 und 1 mM Dithioerythrit euqilibriert war, wurde mit einem linearen Gradienten von 10 - 100 mM Phosphatpuffer und den vorstehend genannten Zusätzen eluiert. Die vereinigten Enzym-enthaltenden Fraktionen wurden durch Ultrafiltration auf 50 ml eingeengt. Diese enthielten 0,37 mg Protein/ml und 26 U/ml Reduktase. Nach 9 h Dialyse gegen 10 mM Tris.HCl pH 7,5, 0,05 % Triton X 100 wurde die Enzymlösung auf eine (R)DEAE-Sephadex A25 Säule mit einem Bettvolumen von 35 ml aufgetragen und das Enzym mit einem linearen Gradienten von 0 - 700 mM KCl in obigem Puffer eluiert. Nach Ultrafiltration der vereinigten Enzym-enthaltenden Fraktionen auf 8,2 ml wurde eine spez. Aktivität von 300 U/mg Protein erhalten (90 U/ml; 0,3 mg Protein/ml).

## Beispiel 4

40 g Bakterienfeuchtmasse Proteus vulgaris wurden mit 60 ml 0,01 M Tris-HCl Puffer pH 7,5 und 1 mM Dithioerythrit aufgeschlämmt, anschießend durch Ultraschallbehandlung (30 min, 40 Watt nicht über 6°C) aufgeschlossen. Das Lysat wurde 10 min. bei 15 000 g zentrifugiert und der Überstand bei 100 000 für 120 min. zentrifugiert. Des Sediment wurde mit 75 ml 0,01 M Tris-HCl pH 7,5 Puffer resuspendiert und mit 1 (v/v) Detergenz (Polyoxoethylene-9-lauryl-ether (Sigma)) versetzt. Nachdem diese Suspension 45 min. im Eisbad gerührt worden war, erfolgte abermals eine Zentrifugation bei 100 000 g für 90 min. Der Überstand enthält die 2-Oxocarbonsäure-Reduktase.

Die Lösung wurde auf eine DEAE-Cellulose-Säule (ø 2,5 cm, Vol. 90 ml) aufgegeben, die mit 0,1 H Tris-HCl pH 7,5, 1 mM Dithioerythrit, 0,8 % Detergenz (v/v), und 4 mM Dithionit äquilibriert worden war. Nachdem mit etwa 2 Säulenvolumina vorstehend genannten Puffers nacheluiert worden war, wurde ein linearer Gradient von 0,1 - 0,7 M KCl (2x225 ml) in obigem Puffer angelegt. Das Enzym eluierte bei etwa 250 mM KCl und war in ca. 60 ml enthalten. Die enzymhaltigen Fraktionen wurden vereinigt und auf eine Hydroxylapatit-Säule (HA Bio-Gel, ø 1,5 cm, Vol. 40 ml), äquilibriert mit 0,01 M Tris-HCl pH 7,5, 1 mM Dithioerythrit, 0,4 (v/v) Detergenz und 4 mM Dithionit, aufgetragen. Nach erfolgter Elution mit vorstehendem Äquilibrierungspuffer (etwa 1 Säulenvolumen) wurde mit einem linearen Gradienten 0 - 150 mM Kaliumphosphat pH 7,5 und vorgenannten Zusätzen das Enzym bei ca. 60 mM Kaliumphosphatpuffer eluiert. Die vereinigten Fraktionen (ca. 30 ml) wurden auf eine zweite DEAE-Cellulose-Säule (ø 1 cm Vol. 20 ml), äquilibriert mit vorstehendem Puffer aufgetragen. Nach kurzer Nachelution wurde die 2-Oxocarbonsäure-Reduktase mit einem linearen Gradienten 0 - 0,4 M KCl wiederum durch obigen Puffer eluiert. Die enzymhaltigen Fraktionen mit der Hauptaktivität wurden vereinigt und durch Ultrafiltration über eine YM 30 Membran (Amicon) auf etwa 6 ml aufkonzentriert. Diese Lösung wurde auf eine Sepharose Cl 6B-Säule (ø 2,5 cm, Vol. 365 ml) äquilibriert mit 0,01 M Tris-HCl Puffer pH 7,5, 0,05 M KCl, und vorgenannten Zusätzen chromatographiert. Das Enzym zeigt eine spezifische Aktivität von 350 U/mg Protein. Beiliegendes Schema gibt den Verlauf der Reinigung wieder.

**Anreichung von 2-Oxocarboxylatreduktase**

| Anreicher-ungsschritt | Volumen V[ml] | Gesamt-protein-menge $M_{Pr}$[mg] | Protein-konzentr. $c_{Pr}$[mg/ml] | Volums-aktivität VA [U/ml] | Gesamt-aktivität GA [U] | Spezif. Aktivität SA [U/mg] | n-fache Anreicher-ung | % Ausbeute |
|---|---|---|---|---|---|---|---|---|
| Rohextrakt Solubilisiertes | 90 | 1750 | 19.4 | 55 | 5000 | 2.8 | – | 100 |
| Enzym nach DEAE- | 80 | 560 | 7 | 50 | 4000 | 7.2 | 2.6 | weitgehend 80 H$_2$ase frei |
| Cellulose 1 nach Hydroxyl | 55 | 248 | 4.5 | 54.5 | 3000 | 12 | 4.3 | 60 H$_2$ase frei |
| apatit nach DEAE- | 40 | 68 | 1.7 | 48 | 1950 | 28.5 | 10.2 | 39 |
| Cellulose 11 u. Ultrafiltration nach Sepharose-CL 6B u. | 6.6 | 10.4 | 1.58 | 166 | 1100 | 105 | 37.5 | 22 |
| Ultrafiltration | 3 | 1.86 | 0.62 | 220 | 660 | 350 | 125 | 13.2 |

**Beispiel 5**

In einer elektrochemischen Zelle wurden 25 ml einer Lösung von 100 mM Kaliumphosphatpuffer pH 7,0 4 mM Methylviologen und 40 mM Phenylpyruvat mit 0,5 mg 2-Oxosäurereduktase gemäß Beispiel 3 bei einer Spannung von -700 mV gegen eine Standardkalomelelektrode reduziert. Der Strom vom 5,8 mA fiel nach 9 h steil ab. Die Reaktionslösung enthielt nach HPLC-Analyse 88 % der erwarteten Phenylmilchsäure. Nach Ansäuren mit verdünnter Schwefelsäure auf pH 1,8 wurde mit Ether extrahiert. Der kristalline Rückstand, 78 % der Theorie, zeigt nach Überführung in das Natriumsalz eine Drehung von $[\alpha]_{589}^{RT}$ = 21,1°; 0,09 mmol/ml H$_2$O. Ein reines Handelspräparat von (2S)-Natriumphenyllactat unter den gleichen Bedingungen gemessen, zeigte einen Wert von -21,4°. Analog wurden (R)-Mandelsäure und (R)-2-Hydroxy-4-mehtylpentanat hergestellt.

Die folgende Tabelle 2 zeigt die relativen Geschwindigkeiten, mit der 2-Oxocarbonsäuren reduziert werden, bezogen auf Phenylpyruvat =1. 3-Oxocarbonsäuren und Hydroxyaceton werden nicht reduziert.

**Tabelle 2**

| Substrat | Relative Geschwindigkeiten P.mirabilis | P.vulgaris | | Relative Geschwindigkeiten P. mirabilis | P.vulgaris |
|---|---|---|---|---|---|
| Phenylpyruvat | =1,00 | =1,00 | 3-Fluorpyruvat | 0,22 | 0,20 |
| Pyruvat | 0,92 | 0,85 | 2-Oxo-4-methylpentanoat | 0,81 | |
| Oxalacetat | 0,73 | 0,50 | (+) 2-Oxo-3methylpentanoat | 0,28 | 0,25 |
| 2-Oxoglutarat | 0,78 | 0,62 | (±) 2-Oxo-3-methylpentanoat | | 0,46 |
| 2-Oxodipat | 0,70 | | Phenylglyoxylat | 0,16 | 0,05 |
| Indolypyruvat | 0,35 | 0,67 | 3-Oxoglutarat | 0 | |
| 5-Benzyloxyindolylpyruvat | 0,30 | | Hydroxyaceton | 0 | |
| 2-Oxopatoat | 0,07 | 0,04 | | | |

**Patentansprüche** für die Vertragsstaaten: BE, CH, DE, FR, GB, IT, LI, NL

1. Pyridinnukleotid-unabhängige und keine Flavingruppen enthaltende 2-Oxocarbonsäurereduktase, erhältlich aus Proteus vulgaris DSM 30118 oder Proteus mirabilis DSM 30115, mit einem Molekulargewicht von etwa 500.000, einem isoelektrischen Punkt bei 4,9 und einem pH-Maximum bei 6,8.

2. Verwendung der 2-Oxocarbonsäurereduktase gemäß Anspruch 1 zur Reduktion von 2-Oxocarbonsäuren und deren Estern.

**Patentansprüche** für den Vertragsstaat: AT

1. Verfahren zur Herstellung einer Pyridinnukleotid-unabhängigen und keine Flavingruppen enthaltenden 2-Oxocarbonsäurereduktase, erhältlich aus Proteus vulgaris DSM 30118 oder Proteus mirabilis DSM 30115, mit einem Molekulargewicht von etwa 500.000, einem isoelektrischen Punkt bei 4,9 und einem pH-Maximum bei 6,8, dadurch gekennzeichnet, daß man einen das Enzym enthaltenden Mikroorganismus aufschließt, den Rohextrakt zentrifugiert, den Überstand ultrazentrifugiert, den Niederschlag in einer Detergenzlösung aufschlämmt und feste Verunreinigungen durch erneute Ultrazentrifugation entfernt.

2. Verwendung der 2-Oxocarbonsäurereduktase gemäß Anspruch 1 zur Reduktion von 2-Oxocarbonsäuren und deren Estern.

**Claims** for the Contracting States: BE, CH, DE, FR, GB, IT, LI, NL

1. A 2-Oxocarboxylic acid reductase which is independent of pyridine nucleotides and does not contain any flavine groups, obtainable from Proteus vulgaris DSM 30118 or Proteus mirabilis DSM 30115 having a molecular weight of about 500,000, an isoelectric point of 4.9 and an optimum pH of 6.8.

2. Use of a 2-oxocarboxylic acid reductase as claimed in claim 1 for the reduction of a 2-oxocarboxylic acid or its esters.

**Claims** for the State: AT

1. A process for the preparation of a 2-oxocarboxylic acid reductase which is independent of pyridine nucleotides and does not contain any fluorine groups, obtainable from Proteus vulgaris DSM 30118 or Proteus mirabilis DSM 30115, having a molecular weight fo about 500,000 an isoelectric point of 4.9 and an optimum pH of 6.8, wherein a micro-organism containing the enzyme is distintegrated, the crude extract is centrifuged, the supernatant liquid is ultracentrifuged, the sediment is suspended in a detergent solution, and solid impurities are removed by further ultracentrifuging.

2. Use of a 2-oxocarboxylic acid reductase as claimed in claim 1 for the reduction of a 2-oxocarboxylic acid or its esters.

**Revendications** pour les Etats Contractants: BE, CH, DE, FR, GB, IT, LI, NL

1. Réductase d'acide 2-oxocarboxylique indépendante de pyridinucléotide et ne contenant pas de groupes flavine, obtenue à partir de Proteus vulgaris DSM 30118 ou Proteus mirabilis DSM 30115, d'un poids moléculaire d'environ 500000, d'un point isoélectrique d'environ 4,9 et d'un pH maximum d'environ 6,8.

2. Utilisation de la réductase d'acide 2-oxocarboxylique selon la revendication 1 pour la réduction d'acides 2-oxocarboxyliques.

**Revendications** four l'Etat Contractant: AT

1. Procédé de préparation d'une réductase d'acide 2-oxocarboxylique indépendante de pyridinucléotide et ne contenant pas de groupes flavine, obtenue à partir de Proteus vulgaris DSM 30118 ou Proteus mirabilis DSM 30115, d'un poids moléculaire d'environ 500000, d'un point isoélectrique d'environ 4,9 et d'un pH maximum d'environ 6,8, caractérisé par le fait qu'on désagrège un microorganisme contenant l'enzyme, on centrifuge l'extrait brut, on ultracentrifuge la couche supérieure, on met en suspension le dépôt dans une solution détergente et on élimine les impuretés solides par nouvelle ultracentrifugation.

2. Utilisation de la réductase d'acide 2-oxocarboxylique selon la revendication 1 pour la réduction d'acides 2-oxocarboxyliques.